# EUROPEAN PATENT APPLICATION

(11) **EP 3 428 618 A1**
(43) Date of publication of application: **16.01.2019**
(21) Application number: 15910186.4
(22) Date of filing: 07.12.2015
(51) Int. Cl.: G01N 21/27

(54) **MANAGEMENT PROGRAM FOR ANALYSIS DEVICE AND MANAGEMENT DEVICE FOR ANALYSIS DEVICE**

(71) Applicant: Shimadzu Corporation, Kyoto-shi, Kyoto 604-8511 (JP)
(72) Inventor: FUJIWARA, Naoya, Kyoto-shi Kyoto 604-8511 (JP); YAMASHITA, Misa, Kyoto-shi Kyoto 604-8511 (JP); HIRAMA, Tomohiro, Kyoto-shi Kyoto 604-8511 (JP); OKADA, Aiko, Kyoto-shi Kyoto 604-8511 (JP)
(74) Representative: Kilian Kilian & Partner
(86) International application number: PCT/JP2015/084327
(87) International publication number: WO 2017/098569

(57) **Abstract**

The present invention provides a management program and apparatus for an analyzing device, that can display descriptions relating to an operation method in a user-friendly manner, even if the number of operation items increases and a display screen is complicated. An analysis system according to the present invention includes an analyzing device 1 and a controller 2 connected to the analyzing device 1. On the controller 2, a general-purpose operating system (OS) is running, and analyzing device control software 11 is executed on the OS. The analyzing device control software 11 and an analyzing device management program 12 are stored in a storage section 10. The analyzing device management program 12 makes a CPU operate as an operation pane display section 13, an explanation pane display section 14, and a distinguishing display section 15.

## Description

### TECHNICAL FIELD

The present invention relates to a program and an apparatus for controlling operations of an analyzing device, such as an infrared microscope, a spectrophotometer, a chromatograph apparatus, or a mass spectrometer, in an analysis using such an analyzing device, and for processing and managing analysis data obtained by such an analyzing device.

### BACKGROUND ART

In recent years, general-purpose personal computers have been used for controlling operations of various analyzing devices, and for processing and managing analysis data obtained by the analyzing devices. In such an analyzing device system, setting various analysis conditions, giving commands (e.g. to start or end an analysis), setting processing conditions for analysis data, preparing a report, and other tasks can be carried out in an operation environment constructed by executing a dedicated program (hereinafter, referred to as "a management program for an analyzing device") installed on a computer.

Information relating to a method of operating a management program for an analyzing device is provided by an operation manual in the form of a booklet or electronic file delivered with the analyzing device. In the operation manual, an operation method, configuration of each section in the analyzing device, maintenance methods, and so on are described using sentences, illustrations, and still images. Users read the description to learn the method of operating the analyzing device. More detailed information for an operation method, troubleshooting and other tasks is also provided using sentences, illustrations, and so on, in the form of online help that can be accessed using the management program for analyzing device. Users can also refer to the description through such online help to learn the operation method.

To help users more easily learn operation methods. Patent Literature 1 discloses a technique that enhances search performance in the operation manual displayed on a display screen so that users can more easily find necessary descriptions, for example. Although the search performance is improved, the description is displayed in the same manner as the description of a conventional operation manual. Therefore, it is difficult for users to comprehend such a description.

### CITATION LIST

### PATENT LITERATURE

Patent Literature 1: JP 2002-278665 A

### SUMMARY OF INVENTION

### TECHNICAL PROBLEM

That is to say, in a recent management program for an analyzing device, the operation items have significantly increased, causing the display on a display screen to be increasingly complicated. Consequently, in a conventional and typical operation manual and online help, it has been difficult for users to comprehend the correspondence between a description in the manual and a component in the actual operation pane. The description relating to a series of operations is serially depicted in the conventional operation manual. With such description, the user is required to repeatedly shift his/her attention between the actual operation pane and a sheet or a displayed area on which the description is shown, each time the user reads a description and performs an operation in the multiple stages of operations. Users might unfortunately not have known how far they have read the description, causing some descriptions to be skipped over. This may lead to an erroneous operation.

In view of the above, the purpose of the present invention is to provide a program and an apparatus for managing an analyzing device that can display descriptions relating to the operation method in an easy-to-understand form for a user even if the operation items increase and the display screen is complicated.

### SOLUTION TO PROBLEM

A management program for an analyzing device according to the present invention developed for solving the previously described problem making a computer connected to an analyzing device function as:
a) an operation pane display section configured to display, on a display screen, an operation pane including a graphic and one or a plurality of input items relating to the graphic;
b) an explanation pane display section configured to display, on the display screen, an explanation pane in which explanation items corresponding to at least one of the input items are listed, the explanation pane being adjacent to the operation pane or superimposed on the operation pane; and
c) a distinguishing display section configured to display, upon indication of one of the explanation items on the explanation pane by a user, the indicated explanation item and the input item in the operation pane which corresponds to the indicated explanation item, in a common manner that is distinguishable from other portions of the explanation pane and other portions in the operation pane.

The "graphic" included in the operation pane is a content produced based on analysis data acquired with an analyzing device, such as a photo image, a map, a spectrum, or a chromatogram. The "input items" included in the operation pane are areas each of which forms a part of the screen display within which users can perform a certain operation using a keyboard, a mouse, or such pointing devices, to give a command to a computer. The input items may individually be an explicitly segmented area, as with the so-called "control" or "button", or it may be an area implicitly set in the graphic. The operation by a user to the input items includes not only an operation of placing a cursor on the area and clicking the area, or performing a predetermined key operation at the position where the cursor is placed. but also an operation of merely placing the cursor. The same applies to an operation upon indicating one of the explanation items on the explanation pane.

Examples of the "common manner" used for the display by the distinguishing display section include: displaying an explanation item and an input item corresponding to the explanation item in the same letter color and background color, displaying those items in the same font (type of characters, boldness, and size), blinking them in the same way, and surrounding them with the same type of frame.

According to the present invention, the explanation item indicated by a user and the input item corresponding to the explanation item can easily be distinguished from other portions of the explanation pane and other portions of the operation pane. Accordingly, users can instantly and instinctively recognize the input item to be operated in the operation pane while reading the explanation item.

It is preferable for the management program for an analyzing device according to the present invention to further include
d) an operation restriction section configured to restrict a user operation to the input item in the operation pane displayed in the common manner to the indicated explanation item.

This configuration prevents the user from erroneously operating an input item which has no relation to the explanation item indicated by the user.

It is preferable for the management program for an analyzing device according to the present invention to further include
e) an operation detection section configured to detect an operation by a user on the input item in the operation pane, in which
the explanation pane display section has, for each of the explanation items, relational information concerning an input item and a corresponding explanation item which relate to a next operation that should be performed after the input item corresponding to the explanation item concerned is operated, and
the distinguishing display section is configured to display, upon the operation detection section detecting a user operation on a certain input item, the explanation item corresponding to the next operation of the certain input item and the input item in the operation pane corresponding to the same explanation item, in a common manner that is distinguishable from the other portions in the explanation pane and other portions of the operation pane.

According to this configuration, when a user operates a certain input item, the explanation item relating to the next operation is displayed. Thus, during the operation, the user only needs to follow the explanation item which is displayed each time one operation is completed. Therefore, a series of operations relating to the analysis result obtained with the analyzing device can quickly be carried out. Furthermore, an erroneous operation that may occur during reading of the description that serially depicts a series of operations, like a conventional operation manual can be prevented. With such a description, a user may lose track of how far the operation with multiple stages has been completed, causing him/her to skip over the description. This may cause the erroneous operation.

A management apparatus for an analyzing device according to the present invention developed for solving the previously described problem includes:
a) a display screen;
b) an operation pane display section configured to display, on the display screen, an operation pane including a graphic and one or a plurality of input items relating to the graphic;
c) an explanation pane display section configured to display, on the display screen, an explanation pane in which explanation items corresponding to at least one of the input items are listed, the explanation pane being adjacent to the operation pane or superimposed on the operation pane; and
d) a distinguishing display section configured to display, upon indication of one of the explanation items on the explanation pane by a user, the indicated explanation item and the input item in the operation pane which corresponds to the indicated explanation item, in a common manner that is distinguishable from other portions of the explanation pane and other portions in the operation pane.

### ADVANTAGEOUS EFFECTS OF INVENTION

According to the present invention, the explanation pane in which explanation items respectively corresponding to the input items are displayed, near an operation pane in which a graphic and the input items relating to the graphic are displayed. The explanation item indicated by a user, and the input item in the operation pane which corresponds to the indicated explanation item, are displayed in a common manner that is distinguishable from other portions of the explanation pane and other portions of the operation pane. Accordingly, a user can instantly and instinctively recognize the input item to be operated.

### BRIEF DESCRIPTION OF DRAWINGS

Fig. 1 is a configuration diagram schematically showing the entirety of an analysis system according to a first embodiment of the present invention.
Fig. 2 is an example of a window that includes an operation pane, and is displayed on a display screen of a controller connected to an infrared microscope that serves as an analyzing device.
Fig. 3 is an example of a window when the display of an explanation pane along with the operation pane is instructed.
Fig. 4 is an example of a window when one of the explanation items displayed in the explanation pane is indicated.
Fig. 5 is a configuration diagram schematically showing the entirety of an analysis system according to a second embodiment of the present invention.
Fig. 6 is a configuration diagram schematically showing the entirety of an analysis system according to a third embodiment of the present invention.
Fig. 7 is an example of a window when an input item included in the operation pane is operated.

### DESCRIPTION OF EMBODIMENTS

Some embodiments of a management program for an analyzing device and a management apparatus for an analyzing device according to the present invention are described hereinafter, with reference to the drawings.

### [First embodiment]

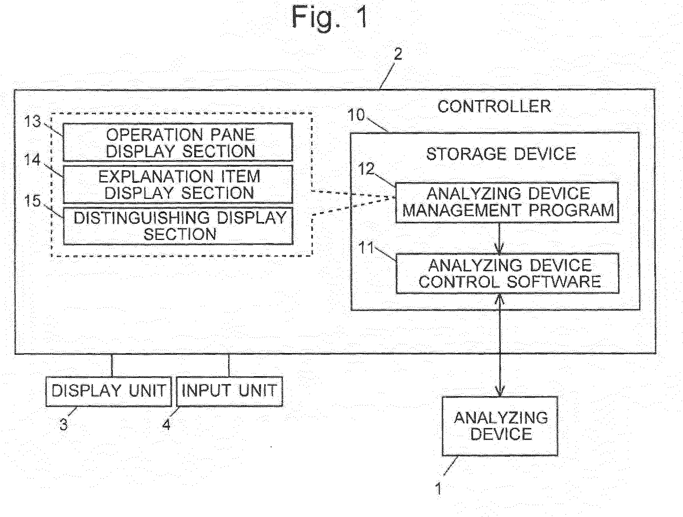

Fig. 1 is a diagram showing the entirety of an analysis system according to the first embodiment. This analysis system includes an analyzing device 1 and a controller 2 connected to the analyzing device 1 via a signal line.

For the analyzing device 1, an image analyzing device, such as an infrared microscope and an electron microscope, as well as a mass spectrometer, a spectrometric analyzing device, and other various analyzing devices can be used. Not only a single analyzing device 1. but also a plurality of analyzing devices 1 may be connected to the controller 2.

The controller 2 controls operations of the analyzing device 1, performs processing and analyzing, and manages analysis results obtained in the analyzing device 1. The controller 2 is actually a general-purpose personal computer (PC) that includes a central processing unit (CPU), a memory, a high-capacity storage device 10 composed of a hard disc drive (HDD) or a solid-state drive (SSD), and so on. On the controller 2, an operating system (OS), such as Windows (registered trademark) for example, is running. On the OS, a piece of analyzing device control software 11 is executed. The analyzing device control software 11 and an analyzing device management program 12 (to be described later) are stored in the storage device 10.

A display unit 3 and an input unit 4 are connected to the controller 2. The display unit 3 includes a liquid crystal display and the like, and displays various kinds of information. The input unit 4 includes a mouse, a keyboard, and the like, and is used by a user to input various commands. In Fig. 1, the display unit 3 and the input unit 4 are depicted outside the controller 2. Here, for example, when the controller 2 is configured by a tablet-type computer, the display unit 3 and the input unit 4 are built inside the controller 2.

The analyzing device management program 12 (hereinafter, it is referred to as a "management program 12") is provided in the controller 2 (i.e., installed on the PC), and acts on the analyzing device control software 11. The management program 12 makes the CPU operate as an operation pane display section 13, an explanation pane display section 14, and a distinguishing display section 15.

Hereinafter, the action of the management program 12 in the analysis system according to the present embodiment is described citing, as an example, a case where the infrared microscope serving as the analyzing device 1 is connected to the controller 2. In this example, it is assumed that the management program 12 is automatically executed after the analyzing device control software 11 is executed for processing and analyzing image data obtained by the infrared microscope. Alternatively, the management program 12 may be executed based on an input of a certain command by a user through the input unit 4.

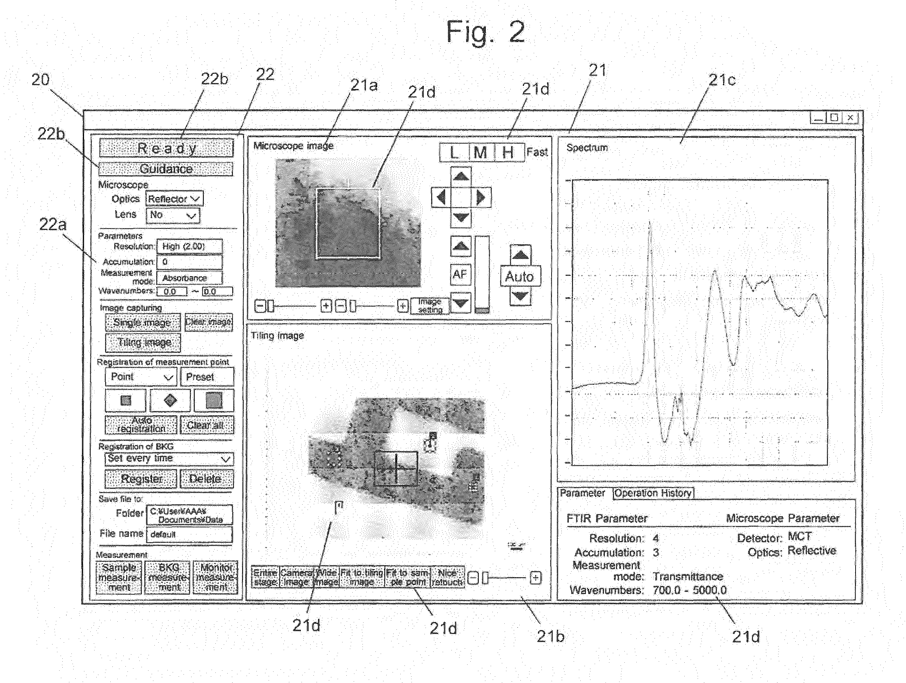

Fig. 2 shows an example of the display screen displayed on the display unit 3 when analysis data obtained by the infrared microscope are processed and analyzed. Upon execution of the analyzing device control software 11, the operation pane display section 1 3 displays an operation pane 20 including a graphic display pane 21 and an input item display pane 22 within the window of the display screen.

The graphic display pane 21 includes: a microscopic image display area 21a for displaying a microscopic image produced based on analysis data obtained with the infrared microscope; a tiling image display area 21b for displaying a tiling image formed by arranging a plurality of microscopic images; and a spectrum display area 21c for displaying a spectrum. A microscopic image, a tiling image, and a spectrum each correspond to a graphic in the present invention. In the display areas 21a, 21b, and 21c, various input items 21d for adjusting the display size of the graphic (scale up and down) and changing display modes are displayed on and around the graphic, in addition to the graphics.

In the input items display screen 22, various input items 22a and various icons 22b are displayed. The input items 22a are to be used for inputting the parameters necessary for the processing and analysis of the graphic, the destination to which the data obtained through the processing and the analysis result are to be stored, and so on. The icons 22b are to be used for instructing the execution of data processing and data analysis, and for displaying the explanation pane 23 that will be described later. The input items 21d and 22a, and icons 22b are each operated through the input unit 4.

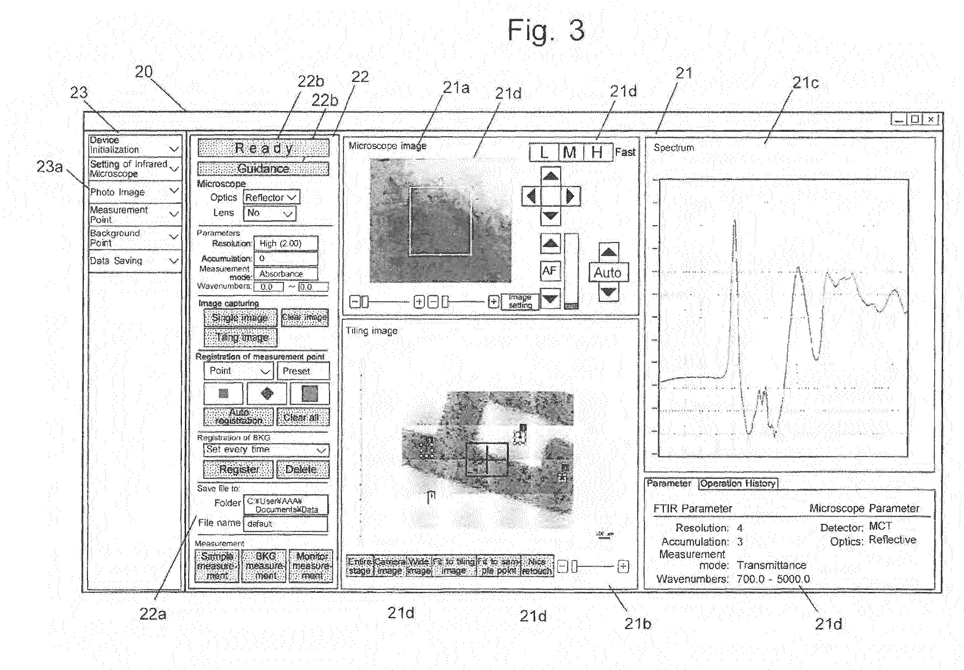

When the display of the explanation pane 23 is instructed (specifically, a "guidance" icon 22b in the input item display pane 22 is clicked) in the operation pane 20 shown in Fig. 2, the explanation pane display section 14 displays the explanation pane 23 next to the operation pane 20 inside the window of the display screen, as shown in Fig. 3. Although the explanation pane 23 is displayed at the left side of the input item display pane 22 in the sheet of Fig. 3, the explanation pane 23 may be displayed at the right side of the graphic display pane 21, or it may also be displayed between the graphic display pane 21 and the input item display pane 22. In addition, the explanation pane 23 may be displayed above or below the operation pane 20. Furthermore, the explanation pane 23 may be displayed in a window that is separate from the window on which the operation pane 20 is displayed, thereby allowing the explanation pane 23 to be superimposed on the operation pane 20.

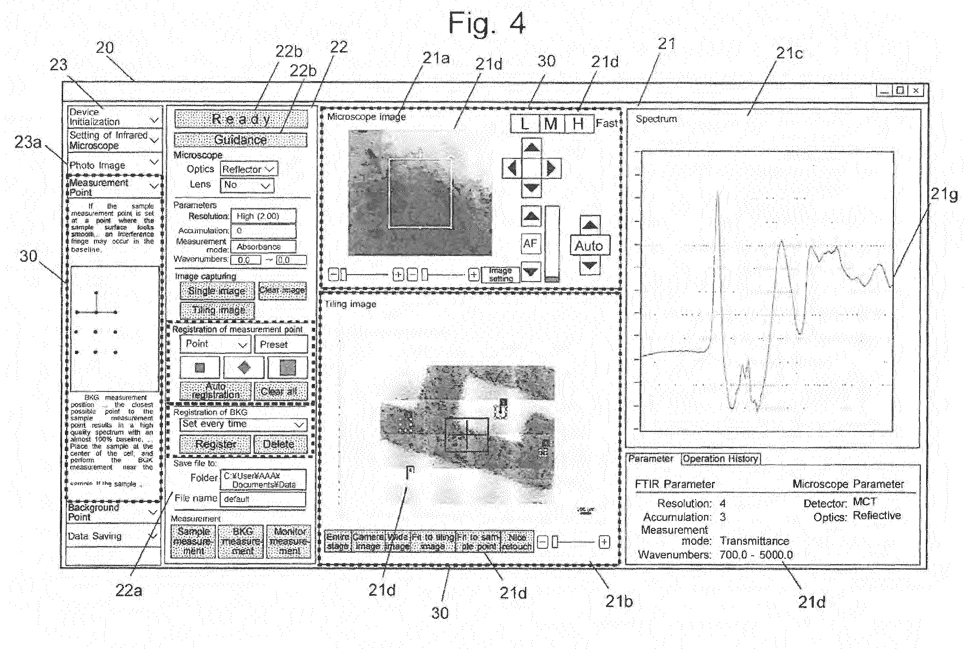

One or a plurality of explanation items 23a corresponding to at least a part of the input items 21d and the input items 22a are displayed on the explanation pane 23. Each of the explanation items 23a includes a title and an explanatory note. When the display of the explanation pane 23 is instructed, only the titles are initially displayed as shown in Fig. 3 (outlined display state). When one of the explanation items 23a is indicated through the input unit 4 under this situation (specifically, when the "V" mark at the right side of each of the titles of the explanation items 23a is clicked), the explanation pane display section 14 switches the explanation item 23a to a detailed display state in which the explanatory note is expanded beneath the title, as shown in Fig. 4. At the same time, the distinguishing display section 15 highlights the portions corresponding to the indicated explanation item 23a among the input items 21d and input items 22a displayed on the operation pane 20 in a manner common to the indicated explanation item. Specifically, the explanation item and the input items which relate to that explanation item are each surrounded by a rectangular broken frame 30. If the input item relating to the explanation item is the input item 21d that is displayed on the graphic display pane 21, an area that includes this input item 21d is surrounded by a rectangular frame 30. Although Fig. 4 shows an example of the explanation item and the corresponding input items which are each surrounded by the rectangular broken frame, the present embodiment is not limited thereto. The explanation items and the input items may be highlighted by other methods, such as changing the background color or letter color of those items, changing the font type of those items, blinking those items, and so on.

As mentioned above, the operation pane 20 for processing and analyzing the analysis data and the explanation pane 23 are displayed side by side in the present embodiment. Thus, a user can operate the input unit 4 for inputting the various input items 21d and 22a on the operation pane 20 while reading the explanation items displayed on the explanation pane 23. In addition, the explanation item 23a indicated by a user and the corresponding input items 21d and 22a are highlighted in the same manner. This enables the user to instantly and instinctively recognize the input items to be operated while reading these explanation items 23a. Furthermore, the explanation items to be displayed on the explanation pane 23 can be switched between the outlined display state and the detailed display state. Thus, a significant amount of information can be displayed on the explanation pane 23.

### [Second embodiment]

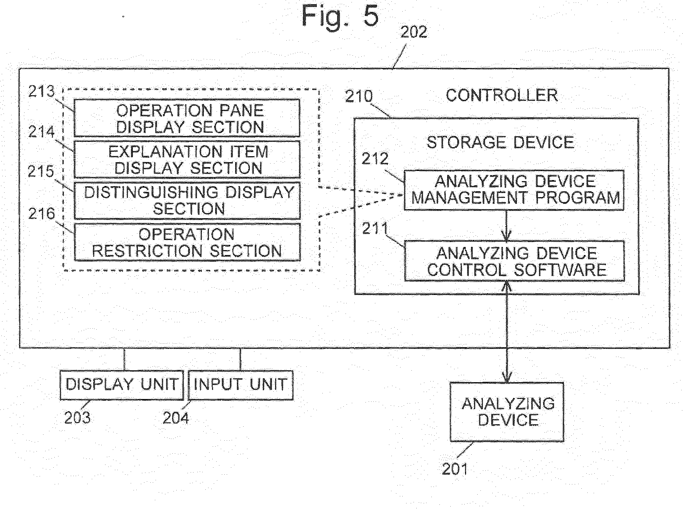

An analysis system according to a second embodiment of the present invention is described as follows, with reference to Fig. 5. It should be noted that structural elements which are identical to or correspond to those in the first embodiment are denoted by numerals whose last two figures and alphabetical letters are the same as those in the first embodiment, and the description thereof is appropriately omitted.

In the analysis system according to the present embodiment, a management program 212 makes a CPU operate as an operation restriction section 216, in addition to an operation pane display section 213, an explanation pane display section 214, and a distinguishing display section 215. When an explanation item 23a in the explanation pane 23 is indicated by a user, the operation restriction section 216 restricts user operations to the input item 21d and the input item 22a which are highlighted in a common manner to the indicated explanation item 23a (see Fig. 4). This can prevent an erroneous operation when a user operates the operation pane while reading explanatory notes.

### [Third embodiment]

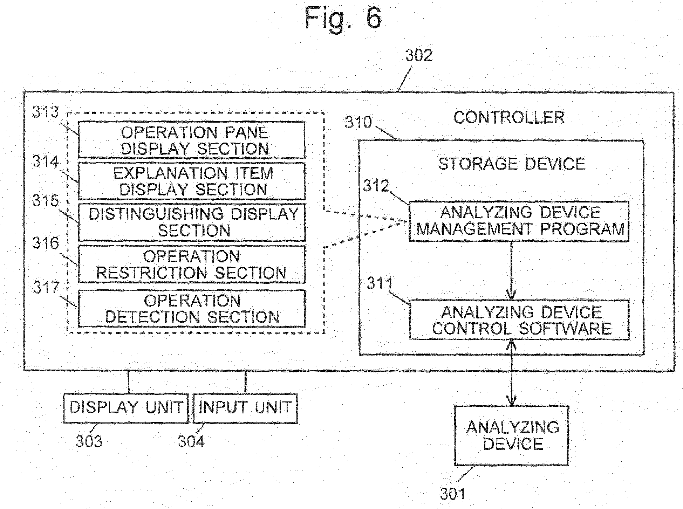

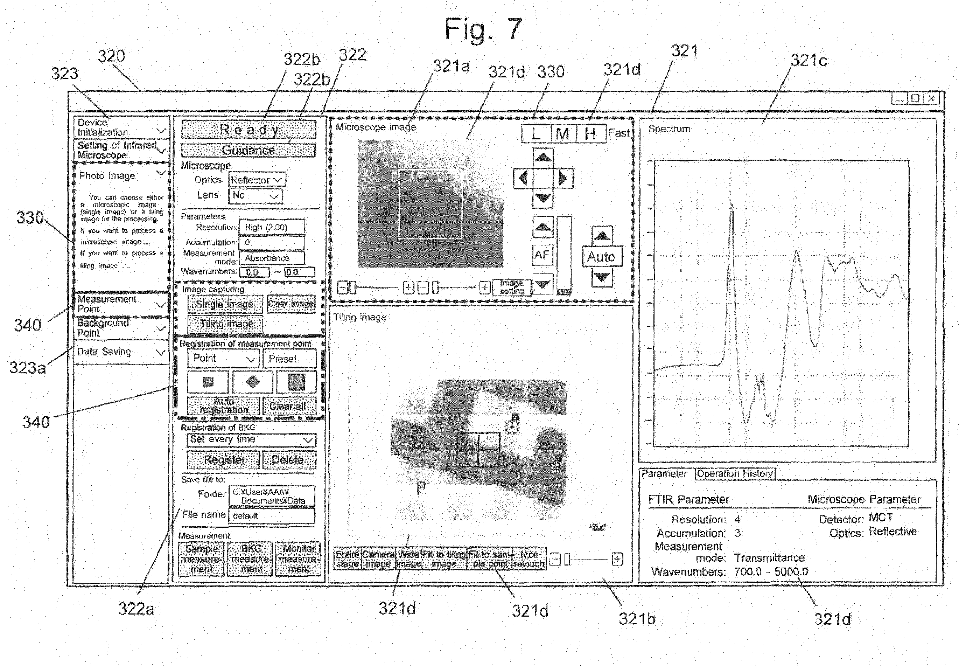

An analysis system according to a third embodiment of the present invention is described as follows, with reference to Figs. 6 and 7. It should be noted that structural elements which are identical to or correspond to those in the second embodiment are denoted by numerals whose last two figures and alphabetical letters are the same as those in the second embodiment, and the description thereof is appropriately omitted.

In the present embodiment, a management program 312 makes a CPU operate as an operation detection section 317, in addition to an operation pane display section 313, an explanation pane display section 314, a distinguishing display section 315, and an operation restriction section 316. The operation detection section 317 can detect an operation, through an input unit 304, relating to an input item 321d and an input item 322a displayed on the operation pane 320. The explanation pane display section 315 has, for each explanation item 323a displayed on the explanation pane 323, relational information concerning an input item and a corresponding explanation item which relate to a next operation that should be performed after an input item corresponding to the explanation item 323a concerned is operated.

Consider the situation in which one of the explanation items 323a in the explanation pane 323 has been indicated by a user and thus is in the detailed display state. In the present embodiment, when a user operates the input item corresponding to that explanation item 323a through the input unit 4, the operation is detected by the operation detection section 317. With this, the distinguishing display section 315 highlights an input item to be operated subsequently to the input item operated by the user and the corresponding explanation item by surrounding each of these items with a rectangular long-dashed-short-dashed frame 340. The example in Fig. 7 shows a window of the display screen when an input item 322a corresponding to the explanation item 323a relating to the "photo image" is operated after this explanation item 323a has been indicated. The explanation item and the input items which relate to the "photo image" are each surrounded by a rectangular broken frame 330. The input item and the corresponding explanation item which relate to the "registration of measurement point", i.e. the operation to be subsequently performed, are each surrounded by a rectangular long-dashed-short-dashed frame 340.

If the title of the explanation item relating to the "registration of measurement point" is indicated in this situation, the explanation item is switched to the detailed display state, and the frames surrounding the explanation item and the input item for highlighting are changed to rectangular broken frames. Meanwhile, the explanation item of the "photo image" is switched to the outlined display state, and the highlighting of the explanation item and the corresponding input items is turned off (see Fig. 4).

As mentioned above, in the present embodiment, a user who has performed an operation relating to a certain input item can at a glance recognize which input item should be subsequently operated. Therefore, a series of the input items necessary for performing the processing and analysis of the analysis data can be quickly set.

### REFERENCE SIGNS LIST

1, 201, 301... Analyzing Device
2, 102, 202... Controller
3, 203, 303... Display Unit
4, 204, 304... Input Unit
10, 210, 310... Storage Device
11, 211, 311... Analyzing Device Control Software
12, 212, 312... Analyzing Device Management Program
13, 213, 313... Operation Pane Display Section
14, 214, 314... Explanation Pane Display Section
15, 215, 315... Distinguishing Display Section
20, 320... Operation Pane
21, 321... Graphic Display Pane
21d, 321d... Input Item
22, 322... Input Item Display Pane
22a, 322a... Input Item
23, 323... Explanation Pane
23a, 323a... Explanation Item
30, 330... Rectangular Broken Frame
340... Rectangular Long-Dashed-Short-Dashed Frame

## Claims

1. A management program for an analyzing device, **characterized by** making a computer connected to an analyzing device function as:
a) an operation pane display section configured to display, on a display screen, an operation pane including a graphic and one or a plurality of input items relating to the graphic;
b) an explanation pane display section configured to display, on the display screen, an explanation pane in which explanation items corresponding to at least one of the input items are listed, the explanation pane being adjacent to the operation pane or superimposed on the operation pane; and
c) a distinguishing display section configured to display, upon indication of one of the explanation items on the explanation pane by a user, the indicated explanation item and the input item in the operation pane which corresponds to the indicated explanation item, in a common manner that is distinguishable from other portions of the explanation pane and other portions in the operation pane.

2. The management program for an analyzing device according to claim 1, wherein
the common manner is one of: changing a letter color or a background color; changing a type of font; blinking; and surrounding an item a frame.

3. The management program for an analyzing device according to claim 1 or 2, further comprising
d) an operation restriction section configured to restrict a user operation to the input item in the operation pane displayed in the common manner to the indicated explanation item.

4. The management program for an analyzing device according to any one of claims 1 to 3, further comprising
e) an operation detection section configured to detect an operation by a user on the input item in the operation pane, wherein
the explanation pane display section has, for each of the explanation items, relational information concerning an input item and a corresponding explanation item which relate to a next operation that should be performed after the input item corresponding to the explanation item concerned is operated, and
the distinguishing display section is configured to display, upon the operation detection section detecting a user operation on a certain input item, the explanation item corresponding to the next operation of the certain input item and the input item in the operation pane corresponding to the same explanation item, in a common manner that is distinguishable from the other portions in the explanation pane and other portions of the operation pane.

5. A management apparatus for an analyzing device comprising:
f) a display screen;
g) an operation pane display section configured to display, on the display screen, an operation pane including a graphic and one or a plurality of input items relating to the graphic;
h) an explanation pane display section configured to display, on the display screen, an explanation pane in which explanation items corresponding to at least one of the input items are listed, the explanation pane being adjacent to the operation pane or superimposed on the operation pane; and
i) a distinguishing display section configured to display, upon indication of one of the explanation items on the explanation pane by a user, the indicated explanation item and the input item in the operation pane which corresponds to the indicated explanation item, in a common manner that is distinguishable from other portions of the explanation pane and other portions in the operation pane.
